# EUROPEAN PATENT SPECIFICATION

(11) **EP 3 470 101 B1**
(45) Date of publication and mention of the grant of the patent: **31.03.2021**
(21) Application number: 17001673.7
(22) Date of filing: 11.10.2017
(51) Int. Cl.: A61M 16/06, A61M 16/08

(54) **RESPIRATORY MASK WITH PIVOTING ELBOW CONNECTOR AND SHROUD**
ATEMMASKE MIT SCHWENKBAREM ELLBOGENVERBINDER UND DECKBAND
MASQUE RESPIRATOIRE DOTÉ D'UN CONNECTEUR COUDÉ PIVOTANT ET D'UN CARÉNAGE

(43) Date of publication of application: 17.04.2019
(73) Proprietor: Air Liquide Medical Systems, 92160 Antony (FR)
(72) Inventor: Masserdotti, Fulvio, 25075 Nave (IT); Bugatti, Ottorino, 25068 Sarezzo (IT); Alberici, Luca, 25030 Roncadelle (BS) (IT)
(74) Representative: Air Liquide

(56) References cited:
- WO-A1-2012/020359
- WO-A1-2016/032343
- WO-A1-2016/206364
- US-A1- 2007 044 804
- US-A1- 2015 328 422
- US-A1- 2017 266 403

## Description

The invention concerns an improved mask body for a respiratory mask, in particular a facial mask covering the nose and mouth of a patient, which is able to cooperate with the upper holding arm carried by a front shroud or retainer, located in front of the mask body, in particular for adult or pediatric uses in the treatment of respiratory conditions or diseases affecting adults, children, toddlers, infants or newborns.

Respiratory masks are commonly used for delivering non-invasive positive pressure ventilation (NPPV) or for continuous positive airway pressure (N-CPAP) therapy in disordered breathing conditions, such as obstructive sleep apnea (OSA), chronic obstructive pulmonary disease (COPD).

In both cases, the mask delivers a flow of breathable gas for, or to assist in, patient respiration. Examples of masks are given by EP-A-462701, EP-A-462701, EP-A-874667, EP-A-1972357 and WO-A-00/57942 as well as WO 2016/206364, US 2015/328422, WO 2012/020359, US 2007/044804, US 2017/266403 and WO 2016/032343.

Such masks typically comprise a rigid or semi-rigid hollow shell, usually made of polymer or silicone, defining a breathing chamber that receives at least a part of the patient's nose in the case of a nasal mask, or both the nose and the mouth of the patient in the case of a facial mask. The hollow shell or body receives the gas from a gas supply line, fixed to an inlet orifice arranged in the mask body by means of a hollow gas connector, for delivering the respiratory gas, such as air under pressure, into the breathing chamber of the shell.

A soft face-contacting cushion comes into contact with the patient's face and conforms to the various facial contours of the patient face thereby ensuring gas tightness. The cushion is usually made of soft, resilient elastomeric material, such as silicone or similar.

The mask may also include a forehead support and further a headgear for correctly positioning, maintaining and/or securing the mask on the head of a patient. The forehead support is usually carried by an upwardly projecting arm that is either directly fixed to the mask body, or to a supplementary front piece, usually called "shroud" or "retainer", that is arranged in front of the mask body, as disclosed by WO-A-0074758, EP-A-1057494 or EP-A-2708258.

When the mask is positioned on the face of a user, such as a patient, the straps of the headgear have to be tightened up so that the mask fits the facial morphology of the user.

This tightening up action may lead to a crushing of the cushion on the nasal bridge region, especially when the upper straps of the headgear of the mask are overly tightened. This may lead to gas leaks and discomfort for the patient. The mask is then less efficient for delivering a gas therapy, which is not acceptable.

In other words, the problem to be solved is to avoid the above problems in providing a mask architecture comprising a mask body and an upwardly-projecting upper arm, and further to allow an easy adjustment of the inclination of the forehead support so that the forehead support can stay in contact with the forehead without changing the position of the cushion on the face/nose of the patient, thereby avoiding or limiting the risks of leaks and discomfort for the patient.

The invention is disclosed in the appended claims.

The solution of the present disclosure concerns a respiratory mask comprising:
- a mask body comprising a gas inlet in fluid communication with an inner chamber, said gas inlet comprising a peripheral border,
- a front shroud comprising a central aperture and an upwardly-projecting upper arm comprising headgear connecting structures, and
- a tubular elbow connector comprising a mask connecting end plugged into the gas inlet of the mask body, said elbow connector comprising an inner passage in fluid communication with the inner chamber of the mask body,
wherein :
- the mask connecting end of the elbow connector and the peripheral border of the gas inlet of the mask body are configured so that the mask connecting end of the elbow connector pivots into the gas inlet of the mask body, and
- the front shroud is fixed to the elbow connector so that the central aperture of the front shroud is a coaxially arranged with the gas inlet of the mask body and with the mask connecting end of the elbow connector.

The mask according to the present invention can further comprise one or more of the followipg additional features:
- the outer peripheral surface of the mask connecting end of the elbow connector and the inner border of the gas inlet form together a ball-and-socket mechanism.
- the elbow connector comprises an annular groove, and the front shroud comprises an annular rim, said annular rim of the front shroud being inserted into the annular groove of the elbow connector, when the front shroud is fixed to the elbow connector.
- the elbow connector further comprises a first abutment, and the front shroud further comprises a second abutment, said second abutment abutting against said first abutment of the elbow connector, when the front shroud is fixed to the elbow connector and the annular rim of the front shroud is inserted into the annular groove of the elbow connector.
- a resilient element is arranged between the mask body and an upper arm of the front shroud so that said resilient element is squeezed between the mask body and the upper arm, when the upper arm pivots toward the mask body.
- the resilient element is arranged on the peripheral outer wall of the mask body.
- the gas inlet of the mask body comprises a collar element arranged around the gas inlet and projecting outwardly, said collar element comprising at least a part of the inner border.
- the collar element comprises an inner arcuated wall comprising at least a part of the inner border of the gas inlet of the mask body.
- a resilient element is arranged on the peripheral outer wall of the mask body.
- the upwardly-projecting upper arm is made of at least one plastic material, such as polycarbonate (PC), polypropylene (PP) or nylon.
- the resilient element is made of resilient material that is able to be deformed, when the upper arm of the front shroud pivots toward the mask body and mechanically acts on the resilient element.
- the resilient material comprises silicone.
- the resilient material has a general tubular shape, such as a cylindrical shape or any other suitable shape allowing a resilient deformation of the resilient element, when pushed toward/against the mask body by a pivoting or bending motion of the upper arm with respect of and toward the mask body.
- said at least one resilient element has a hollow structure, such as a tubular-structure, for instance a hemi-cylinder structure.
- the front shroud comprises a central aperture coaxially arranged with the gas inlet of the mask body.
- the central aperture of the front shroud and the gas inlet of the mask body are traversed by the hollow connector.
- the hollow connector is a curved connector, namely an elbow connector.
- the mask connecting end of the elbow connector and the inner border of the gas inlet of the mask body are configured for allowing a pivoting of the mask connecting end of the elbow connector into the gas inlet of the mask body of an angle α of between about 0° and 30°, preferably less than 20°.
- the elbow connector is snap-fitted into the gas inlet of the mask body.
- the front shroud further comprises two lateral arms projecting laterally.
- the front shroud and/or the two lateral arms are made of at least one plastic material, i.e., polymer material, such as polycarbonate (PC), polypropylene (PP) or nylon.
- the two lateral arms comprise headgear connecting structures, such as slots, hooks and/or similar.
- the respiratory mask further comprises a flexible cushion fixed to the mask body.
- the front shroud is mounted in front of the mask body, i.e. surrounding, enveloping or wrapping at least a part of the peripheral outer surface of the mask body.
- the front shroud is held integrally with the mask body by the hollow connector. The front shroud is sandwiched between the hollow connector and the mask body.
- the respiratory mask further comprises a headgear fixed to the headgear connecting structures of the front shroud.
- the headgear comprises straps, preferably made of fabric material.
- the respiratory mask is a facial mask.
- the elbow connector comprises an upstream and a downstream linear portions separated by a curved portion, i.e. a bent portion.
- the downstream portion of the elbow connector, which is also called the mask connecting end, is snap-fitted into the gas inlet of the mask body.
- the downstream portion or mask connecting end of the elbow connector comprises a tubular body traversed by the inner passage and a tubular sleeve is arranged around the tubular body, said tubular sleeve comprising a free open end and a blind end, and delimits a venting passage between the tubular sleeve and the tubular body, the free open end of the tubular sleeve being arranged around the outlet orifice of the tubular body, said tubular sleeve being fixed, by the blind end, to the peripheral outer wall of the tubular body, said blind end of the tubular sleeve comprising a plurality of venting holes putting the venting passage in fluid communication with the atmosphere. Preferably the venting holes are arranged in a circular arc. The venting ports, which are orifices that allow the venting of CO₂-enriched gas to the atmosphere, i.e. gas expired the patient that wears the mask. Preferably, the venting ports have a generally tronconical shape and preferably a size, such as an outlet diameter, of between about 0,3 mm and 3 mm, i.e. measured at their exit on the side of the surrounding atmosphere.
- the elbow connector is designed and adapted for conveying a gas, such as air under pressure (i.e. > 1 bar).
- the elbow connector has an inner diameter of the inner gas passage of between 10 mm and 30 mm, measured at the outlet orifice.
- the respiratory mask further comprises an anti-asphyxia system (AAS) comprising a venting port cooperating with a mobile flap. Such an AAS is known from US-A-5438981 and WO-A-00/38772. Such AAS are useful in facial masks in case of failure of the gas delivery apparatus.
- the mask body is a hollow body, also called mask shell, comprising an inner chamber and a gas inlet orifice in fluid communication with said inner chamber for allowing gas entering into said inner chamber by said inlet orifice.
- the gas inlet orifice of the mask hollow body has a diameter of about between 1 and 3 cm.
- the gas inlet orifice is arranged substantially centrally in the mask body.
- the mask body has a generally tridimensional (3D) shape, preferably having a general contour that is substantially triangular or trapezoidal.
- when the elbow connector is fluidly connected to the mask hollow body, the inner chamber of the mask body is in fluid communication with the ambient atmosphere through the venting passage and the venting ports of the hollow tubular connector.
- the tubular connector comprises a tubular upstream portion for receiving and securing thereto a hose or gas line by means of a connecting piece, such as intermediary connector that is rotatable with respect to the elbow connector.
- when the tubular elbow connector is inserted into the gas inlet orifice of the mask body, the gas passage traversing the hollow connector is in fluid communication with the inner chamber of the mask body for delivering respiratory gas therein, whereas said inner chamber of the mask body is further in fluid communication with the venting passage and with the venting holes of the elbow connector of the invention for venting expired-gases to the ambient atmosphere, i.e. out of the mask's inner chamber.
- the flexible cushion is fixed to the mask body, for instance the cushion can be snap-fitted to the mask body, or fixed thereto by any other means, such as glued to it or even molded in one piece with the mask body.
- the cushion can be either a nasal cushion receiving only the nose of the patient (i.e. in the case of a nasal mask), or a facial cushion receiving both the nose and the mouth of the patient (i.e. in the case of a facial mask).
- the respiratory mask is preferably a facial mask covering, in use, the nose and the mouth of the patient.
- preferably, the cushion is a facial cushion configured and sized for coming into contact, when the mask is worn by a user, i.e. a patient, with specific regions of the user's face, comprising a nasal bridge region including lateral nose regions, a chin region, i.e. the area located under the lower lip, and cheek regions located on each lateral sizes of the nose and mouth, said cheek regions linking the nasal bridge region including lateral nose regions to the chin region. The nasal bridge region can be an area of the nose located either at the junction of bone and cartilage, or below or above said bone/cartilage junction of the nose; the chin region is expending from the lower lip to the chin, whereas the cheek regions are the areas of the face located on right and left sizes of the nose and mouth.
- the cushion comprises a cushion body delimiting a respiratory chamber comprising, on the one hand, a front aperture (i.e. front opening) adapted for receiving at least part of the patient's nose and/or mouth, in use, i.e., when the patient wears the mask, especially a facial mask receiving both the patient's nose and mouth, and/or, on the other hand, a rear aperture (i.e. rear opening) that opens into the inner chamber of the mask body, when the cushion is firmly fixed to the mask body, so that the respiratory chamber of the cushion is in fluid communication with the inner chamber of the mask body.
- the cushion has a generally tridimensional (3D) shape, preferably a 3D saddle, triangular or trapezoidal shape.
- the front aperture (i.e. front opening) of the cushion is configured and sized for receiving at least part of the patient's nose and/or mouth, in use.
- the cushion comprises at least one membrane forming a kind of flexible skirt along the cushion aperture receiving the patient's face, i.e. nose and/or mouth.
- the cushion comprises a membrane forming a flexible skirt along the cushion aperture that deforms so as to match the contours of the face of the patient thereby ensuring a gas tightness when the patient inserts his/her face, i.e. nose or nose and mouth, into the front aperture of said cushion.
- in another embodiment, the flexible cushion can comprise several membranes, e.g. two or three superimposed membranes that ensure gas tightness, the outer membrane being in contact with the patient's face, when the patient wears the mask.
- the one or several membranes forming a flexible skirt are arranged around all along the periphery of the front aperture of the respiratory chamber of the cushion.
- the cushion is made of a soft flexible material, preferably silicone or similar.
- the headgear connecting structures of the front shroud comprise hooks, slots, snap-fit connectors or similar, including combination thereof.
- the front shroud comprises an upwardly-projecting upper arm and two lateral arms projecting laterally, said upwardly-projecting upper arm facing, i.e. in use, the nose and part of the forehead of the patient, and said two lateral arms projecting laterally, i.e. in use, along part of the cheeks of the patient.
- the two lateral arms and/or the upwardly-projecting upper arm are preferably integrally fixed to the front shroud, for instance molded in one-piece or glued together.
- the upwardly-projecting upper arm is also called a holding arm.
- the two lateral arms and/or the upwardly-projecting upper arm each comprises a free distal end carrying the headgear connecting structures.
- a headgear comprising several straps is fixed to the headgear connecting structures of the two lateral arms and/or of the upwardly-projecting upper arm.
- the straps of the headgear are made of fabric and/or polymer materials, or the like.
- the upwardly-projecting upper arm and/or of the two lateral arms have an elongated shape, for example a band or ribbon shape. Of course, other shapes are possible.
- the upwardly-projecting upper arm has a length of about 2 and 8 cm.
- each lateral arm has a length of about 3 and 6 cm.
- at least a region of the upwardly-projecting upper arm and/or of the two lateral arms is configured or shaped so as to spouse, i.e. to match, the external profile, i.e. the outer contours, of the mask body, when the front shroud is positioned in contact to the mask body. Preferably, they have a curved-shape.
- the elbow connector is fixed to the body mask so as to be in fluid communication with one another thereby allowing gas flows to circulate, in normal use:
   - from the elbow connector to the inner chamber of the mask body during the inspiration phases of the patient, when air under pressure is delivered to the patient,
   - and vice versa, i.e. from the mask body to the elbow connector during expiration phases, when the patient exhales CO₂-enriched gases that have to be vented to the atmosphere through the venting passage and venting holes of the tubular sleeve arranged around the tubular body of the hollow connector.
- the elbow connector is fixed to and rotatable with respect to the mask body.

The invention also concerns an assembly for delivering a gas to a patient comprising a gas delivery device, such as a medical ventilator, and a respiratory mask according to the present invention. Preferably the gas delivery device is fluidly connected to the respiratory mask by means of a gas line, such as a flexible hose.

An embodiment of a respiratory mask according to the present invention is shown in the enclosed illustrative, but not limitative, Figures, among which:
- Figure 1 represents a general view of an embodiment of a mask according to the present invention,
- Figure 2 is a cross sectional view the mask of Figure 1, and
- Figure 3 is an enlarged view of the connecting structures of the mask of Figure 2.

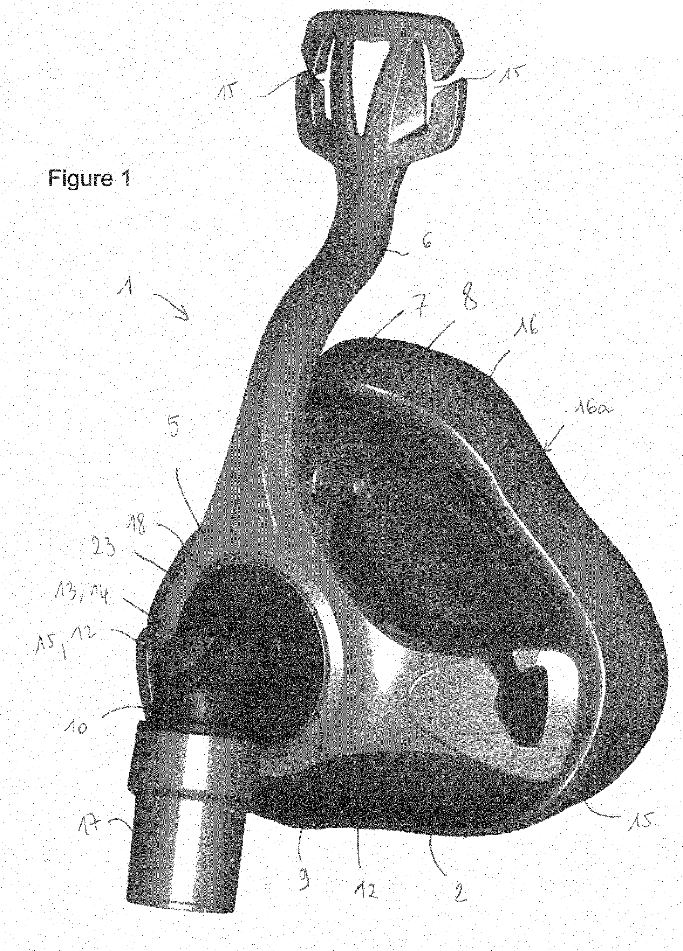

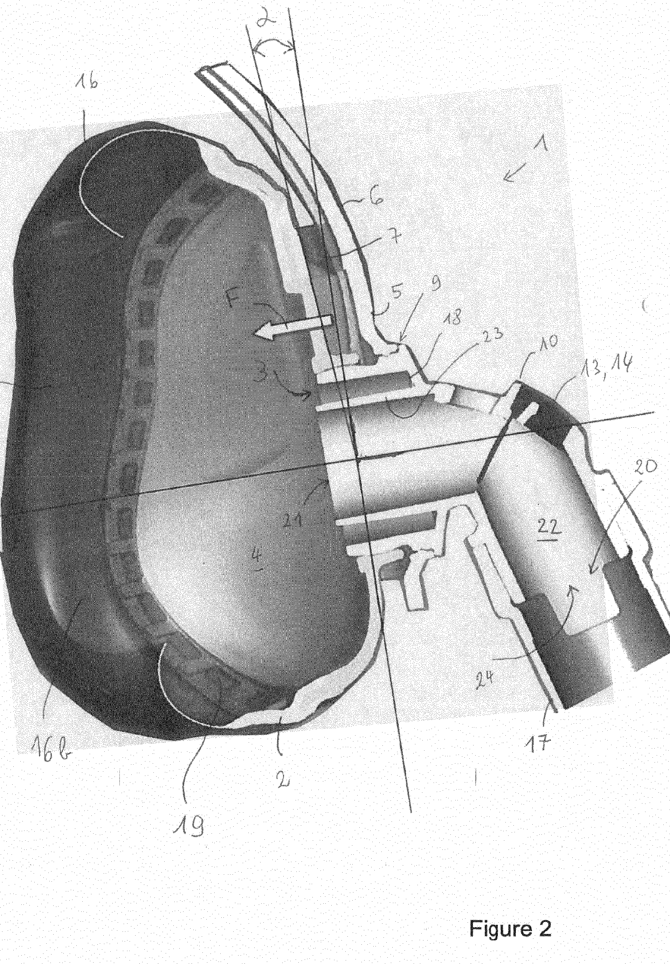

Figure 1 represent general view of an embodiment of a respiratory mask 1, namely a facial mask, according to the present invention, comprising a hollow curved connector 10, a mask body or shell 2 defining an inner chamber 4 (Fig. 2) comprising a gas inlet 3 in fluid communication with said inner chamber 4, and a front shroud 5, called "retainer", "shroud" or "front piece".

The front shroud 5 comprises two lateral arms 12 and a upper arm 6 that are integrally fixed to or molded in one-piece with the rest of the front shroud 5.

When the front shroud 5 is fixed to the mask body 2, the upper arm 6 projects upwardly, i.e. about vertically, with respect to the mask body 2, whereas the two lateral arms 12 projecting laterally from said mask body 2 in opposite directions, i.e. one toward the right side of the mask 1 and the other toward the left side of the mask 1.

The two lateral arms 12 and the upper arm 6 have free distal ends carrying headgear connecting structures 15, such as slots, hooks or the like, so as to fix a headgear thereto (not shown). A headgear typically comprises several straps of fabric and/or polymer materials, or the like, and is used for securing and maintaining the mask 1 in position on the patient's face, when the mask is used, i.e. worn by the patient.

The upper arm 6 and/or of the two lateral arms 12 have typically elongated shapes, for example band or ribbon shapes, and are made of a material, such as a polymer material, e.g. polycarbonate (PC), polypropylene (PP) or nylon so as to be slightly bendable toward the patient's face under the forces applied by the headgear, when said headgear is adjusted to the morphology of the patient's head. The front shroud 5 can be made of the same polymer material or of any other suitable material.

The shroud 5 further comprises a central aperture 9 that is traversed by part of the hollow connector 10, as illustrated in Figure 2.

Furthermore, the mask body 2 can be made from a unique piece or from several pieces or sub-units that are subsequently assembled together and made integral by means of any available fixing technique, for instance they can be glued, thermo-welded or similarly affixed together. It can be made of a polymer or plastic material, such as polypropylene (PP), polycarbonate (PC) and nylon, or similar.

Further, the facial mask 1 of the present invention also comprises a flexible cushion 16 that is fixed to the rear side of the mask body 2. Typically, the flexible cushion 16 is a tridimensional hollow structure forming an inner respiratory chamber with a front aperture 16a adapted, i.e. conformed and sized, for receiving at least part of the nose and mouth of the patient as above explained, when the patient wears the mask, so that the patient can breathe the respiratory gas contained in the inner chamber 4 of the mask body 2 and/or in the respiratory chamber of the cushion 16 as those chambers are in fluid communication with one another.

Indeed, the cushion 16 comprises a cushion body delimiting the respiratory chamber 16b and further comprises a rear aperture or rear opening 16a delimited by a rear border that opens into the inner chamber 4 of the mask body 2, when the cushion 16 is firmly fixed to the mask body 2, so that gas can freely pass from the inner chamber 4 of the mask body 2 towards the respiratory chamber 16b of the cushion 16, and vice versa.

The inner chamber 4 of the mask body 2 and the respiratory chamber of the cushion 16 form together a large inner compartment 16b, 4, wherein the patient can inspire fresh gas, such as air, during inhalation phases, and exhale CO₂-enriched gas, during expiration phases.

In other words, the respiratory chamber 16b of the cushion 16 and the inner chamber 4 of the mask body 2 are both in fluid communication with the central gas passage 22, i.e. the lumen, of the elbow connector 10 so that a gas flow can freely travel from said tubular connector 10 to said chambers, or vice versa.

In order to provide efficient gas tightness (i.e. seal) and/or increased comfort for the patient, the aperture 16a of the cushion 16 is delimited by a flexible membrane that comes into contact with the patient's face and conforms with his/her facial morphology, when the mask 1 is worn by the patient. This membrane constitutes a kind of soft flexible skirt delimiting the front aperture of the cushion 16. Of course, depending on the embodiment, two or more superimposed membranes can also be used as using several membranes may improve gas tightness in certain circumstances, e.g. for some particular patient's morphologies.

The cushion 16 has, in the present case, a generally tridimensional (3D) trapezoidal shape, but other shapes are also possible such as triangular or saddle shapes, so as to better fit with the contours of the patient's face, especially in the nasal and mouth regions. When the facial mask 1 is worn by the patient, i.e. when the front aperture 16a of cushion 16 receives at least a part of the nose and the mouth of the patient, i.e., when the patient introduces his/her nose into the internal volume defined by the large inner compartment 16b, 4, and breathes the gas contained therein or exhales CO₂-enriched gases into it, the cushion 16 and the membrane forming the peripheral border or skirt of the front aperture of the cushion 16 are in contact with particular and well defined regions of the patient's face, thereby ensuring gas tightness.

Preferably, the cushion 16 is a facial cushion configured and sized for coming into contact, when the mask is worn by a user, with at least the nasal bridge region (including lateral nose regions), the chin region, i.e. the area located under the lower lip, and the cheek regions located on both lateral sizes of the nose and mouth of the patient, said cheek regions linking the nasal bridge region including lateral nose regions to the chin region. The nasal bridge region can be an area of the nose located either at the junction of bone and cartilage, or below or above said bone/cartilage junction of the nose; the chin region is expending from the lower lip to the chin, whereas the cheek regions are the opposite areas located on right and left sizes of the nose and mouth. Of course, other embodiments and shapes of the cushion 16 are possible.

The cushion 16 (i.e., including cushion body and membrane) is preferably made of a flexible and soft material, such as silicone or the like. Preferably, the membrane and the cushion body are molded in one piece. The cushion 16 is fixed to the mask body 2 by means of a male/female connecting system 19, such as male/female connections, that are configured or shaped so as to fit together (see Fig. 2) for ensuring for instance a snap-fit connection, a "sandwich-type connection or the like. They can also be glued together or the like.

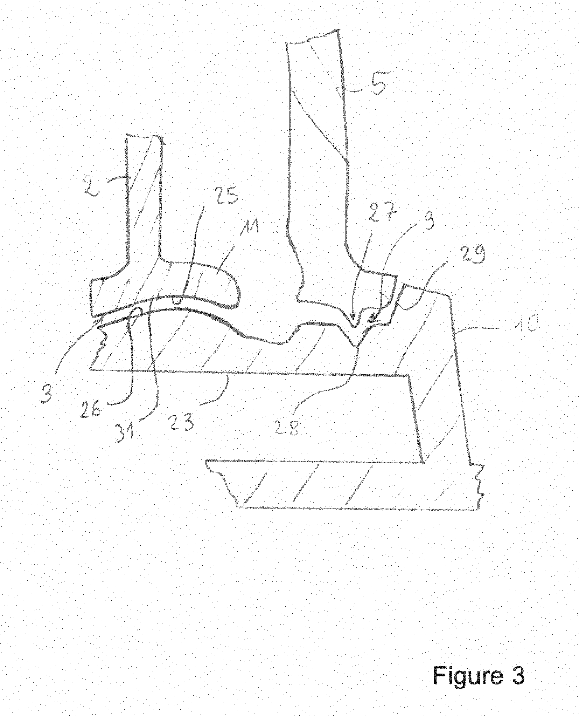

The gas inlet 3 of the mask body 2 is designed for fixing thereto the tubular gas connector 10 as shown in Fig. 2 and 3. Further, the front shroud 5 is carried by the tubular connector 10, whereas the connector 10 is plugged into the mask body 2.

The hollow connector 10 has a tubular body traversed by an inner passage 22 comprising an inlet 20 and outlet 21 orifices. The outlet 21 orifice of the hollow connector 10 is arranged on the mask connecting end 23 of the hollow connector 10, whereas the gas feeding end 24 of the hollow connector 10 comprises the inlet 20 of the hollow connector 10. The tubular body of the hollow elbow connector 10 is curved so as to have a L- or elbow-shape.

In the case of a facial mask, it is wise that the connector 1 further comprises an anti-asphyxia system comprising a venting port 13 cooperating with a mobile flap 14, i.e. a flexible flap, so as to allow the patient breathing fresh air through the venting port 13 in case of failure of the gas source, such as a medical ventilator. Such anti-asphyxia systems are known in the art, as disclosed by US-A-5438981 or WO-A-00/38772.

The upstream portion of the hollow connector 10, that forms the gas feeding end 24 of the hollow connector 10, is fed with gas, such as pressurized air, by a flexible hose (not shown) or the like conveying the gas delivered by a medical gas source, such as a medical ventilator or apparatus. The flexible hose is fluidly connected to the hollow connector 10 by means of an intermediary connector 17, i.e. a tubing element, that is preferably rotatable with respect to the hollow curved connector 10.

In the embodiment shown in Fig. 1 and 2, the curved connector 10 further comprises a gas venting system comprising venting passage(s) and venting orifice(s) 18 or port(s) for recovering CO₂-riched expired gases exhaled by the patient and for venting them to the ambient atmosphere.

The hollow connector 10 is entirely made of a plastic material, such as PP, PC or nylon. It is fluidly connected to the mask body 2 for feeding gas into the inner chamber 4. In other words, the gas that passes through the inner passage 22 of the hollow curved connector 10 is subsequently delivered into the inner chamber 4 of the mask body 2 for being inhaled by a patient wearing the mask 1.

The front piece or shroud 5 comprises a central aperture 9 or opening, as shown in Fig. 2, and several headgear connecting structures 15, such as hooks and/or slots or similar, for receiving the straps of a headgear (Fig. 1). The headgear connecting structures 15 are arranged at the free ends of the lateral arms 12 and the upper arm 6.

The gas inlet 3 of the mask body 2 and the central aperture 9 of the front shroud 5 are coaxially traversed by the hollow connector 1, as shown in Fig. 2.

The elbow connector 10 can be snap-fitted into the gas inlet 4 of the mask body 2 thereby prohibiting any undesired withdrawal of the hollow connector 10 from the mask body 2.

In other words, the respiratory mask 1 comprises:
- a mask body 2 traversed by a gas inlet 3 having an inner border 25, said gas inlet 3 being is in fluid communication with the inner chamber 4,
- a front shroud 5 comprising a central aperture 9 and an upwardly-projecting upper arm 6 comprising headgear connecting structures 15, and
- a tubular elbow connector 10 comprising a mask connecting end 23 plugged into the gas inlet 3 of the mask body 2, said elbow connector 10 comprising an inner passage 22 in fluid communication with the inner chamber 4 of the mask body 2 so that gas conveyed by the inner passage 22 of said elbow connector 10 can be delivered to the inner chamber 4 of the mask and afterwards inhaled by the patient.

Further, according to the present invention, the mask connecting end 23 of the elbow connector 10 and the inner border 25 of the gas inlet 3 of the mask body 2 are configured so that the mask connecting end 23 of the elbow connector 10 pivots into the gas inlet 3 of the mask body 2, when the tubular elbow connector 10 is plugged into the gas inlet 3 of the mask body 2. The front shroud 5 is further fixed to the elbow connector 10 so that the central aperture 9 of the front shroud 5 is a coaxially arranged with the gas inlet 3 of the mask body 2 and, at the same time, with the mask connecting end 23 of the elbow connector 10.

Said pivoting motion is obtained thanks to the outer peripheral surface 26 of the mask connecting end 23 of the elbow connector 10 and the inner border 25 of the gas inlet 3 that constitute a ball-and-socket mechanism 25, 26, as shown in Figures 2 and 3.

Furthermore, as detailed in Fig. 3, for ensuring an efficient fixation of the front shroud 5 to the elbow connector 10, an annular groove 28 is arranged on the elbow connector 10, namely around its peripheral outer surface, whereas an annular rim 27 is arranged in the front shroud 5, namely on the inner wall surface of the central aperture 9, so that the annular rim 27 of the front shroud is lodged, i.e. inserted, into the annular groove 28 of the elbow connector 10, when the front shroud 5 is integrally fixed to the elbow connector 10.

For limiting the translation course of the shroud 5, while it is positioned around the elbow connector 10, it is provided a first abutment 29 on the elbow connector 10, and further a second abutment 30 on the front shroud 5 that abut one against the other, when the front shroud 5 is inserted around the elbow connector 10 so that the annular rim 27 of the front shroud 5 can be well-inserted into the annular groove 28 of the elbow connector 10. Preferably, the first abutment 29 and second abutment 30 have ring or collar shapes as shown in the embodiment of Fig. 2 and 3, or the like.

Thanks to the ball-and-socket mechanism 25, 26, the front shroud 5 can pivot toward the mask (see arrow F in Fig. 2), and vice-versa, with respect to the axis of the mask connecting end 23 or portion of the elbow connector 10, preferably of an angle α of less than 30°, typically less 20°, as represented in Fig. 2.

In the aim of limiting said pivoting motion, a resilient element(s) 7 is(are) arranged between the mask body 2 and the upper arm 6 of the front shroud 5 as shown in Fig. 2. Said resilient element 7 is squeezed between the mask body 2 and the rear surface or side of the upper arm 6 of the front shroud 5, when the upper arm 6 pivots toward the mask body 2, for instance when the headgear is adjusted to the facial morphology of a user.

The resilient element 7 is arranged, e. g. glued or over-molded, on the peripheral outer wall 8 of the mask body 2. It is made of resilient material, such as silicone or the like, that is able to be deformed when the upper arm 6 of the front shroud 5 mechanically acts on it. Actually, the resilient element 7 acts as a soft bumper that limits the course of the upper arm 6, when it pivots toward the mask body 2, when someone pulls on the straps of the headgear for adjusting said headgear to the morphology of the patient's face. The resilient element 7 can have any suitable shape, and can be made of a unique piece or unit or of two or more sub-units or several pieces, that have a same shape or different shapes. It further can have a hollow structure or a non-hollow structure, i.e. a solid structure, for instance made of little block or piece of resilient material. Furthermore, the resilient element 7 can be removably attached to the mask body 2 or integrally fixed to the body 2, i.e. in a non-removable manner.

Generally speaking, the respiratory mask 1 of the present invention, such as a facial mask, is light, comfortable to wear, easy to position and secure on the patient's face, and provide a good tightness, i.e. gas seal.

The respiratory mask of the present disclosure can be used in a method for treatment of a respiratory disorder or condition affecting infant, toddler and child patients as well as adult patients.

## Claims

1. Respiratory mask (1) comprising:
- a mask body (2) comprising a gas inlet (3) in fluid communication with an inner chamber (4), said gas inlet (3) comprising an inner border (25),
- a front shroud (5) comprising a central aperture (9) and an upwardly-projecting upper arm (6) comprising headgear connecting structures (15), and
- a tubular elbow connector (10) comprising a mask connecting end (23) plugged into the gas inlet (3) of the mask body (2), said elbow connector (10) comprising an inner passage (22) in fluid communication with the inner chamber (4) of the mask body (2),
wherein :
- the mask connecting end (23) of the elbow connector (10) and the inner border (25) of the gas inlet (3) of the mask body (2) are configured so that the mask connecting end (23) of the elbow connector (10) pivots into the gas inlet (3) of the mask body (2), and
- the front shroud (5) is fixed to the elbow connector (10) so that the central aperture (9) of the front shroud (5) is a coaxially arranged with the gas inlet (3) of the mask body (2) and with the mask connecting end (23) of the elbow connector (10)
**characterized in that**:
- the elbow connector (10) comprises an annular groove (28), and
the front shroud (5) comprises an annular rim (27), said annular rim (27) of the front shroud (5) being inserted into the annular groove (28) of the elbow connector (10), when the front shroud (5) is fixed to the elbow connector (10),
- the elbow connector (10) further comprises a first abutment (29), and the front shroud (5) further comprises a second abutment (30), said second abutment (30) abutting against said first abutment (29) of the elbow connector (10), when the front shroud (5) is fixed to the elbow connector (10) and the annular rim (27) of the front shroud (5) is inserted into the annular groove (28) of the elbow connector (10), and
- a resilient element (7) is arranged between the mask body (2) and an upper arm (6) of the front shroud (5) so that said resilient element (7) is squeezed between the mask body (2) and the upper arm (6), when the upper arm (6) pivots toward the mask body (2).

2. Respiratory mask according to the preceding claim, **characterized in that** the outer peripheral surface (26) of the mask connecting end (23) of the elbow connector (10) and the inner border (25) of the gas inlet (3) form together a ball-and-socket mechanism (25, 26).

3. Respiratory mask according to anyone of the preceding claims, **characterized in that** the resilient element (7) is arranged on the peripheral outer wall (8) of the mask body (2).

4. Respiratory mask according to anyone of the preceding claims, **characterized in that** the gas inlet (3) of the mask body (2) comprises a collar element (11) arranged around the gas inlet (3) and projecting outwardly, said collar element (11) comprising at least a part of the inner border (25).

5. Respiratory mask according to anyone of the preceding claims, **characterized in that** the collar element (11) comprises an inner arcuated wall (31) comprising at least a part of the inner border (25) of the gas inlet (3) of the mask body (2).

6. Respiratory mask according to anyone of the preceding claims, **characterized in that** the front shroud (5) further comprises two lateral arms (12) projecting laterally.

7. Respiratory mask according to anyone of the preceding claims, **characterized in that** the two lateral arms (12) comprise headgear connecting structures (15), preferably a headgear is fixed to the headgear connecting structures (15) of the front shroud (5).

8. Respiratory mask according to anyone of the preceding claims, **characterized in that** the front shroud (5) comprising the upwardly-projecting upper arm (6) and/or the two lateral arms, is made of at least one polymer material.

9. Respiratory mask according to anyone of the claims 1 to 7, **characterized in that** it further comprises a flexible cushion (16) fixed to the mask body (2).

10. Respiratory mask according to anyone of the preceding claims, **characterized in that** the front shroud (5) is held integrally on the hollow connector (10).

11. Respiratory mask according to anyone of the preceding claims, **characterized in that** the mask connecting end (23) of the elbow connector (10) and the inner border (25) of the gas inlet (3) of the mask body (2) are configured for allowing a pivoting of the mask connecting end (23) of the elbow connector (10) into the gas inlet (3) of the mask body (2) of an angle α of between 0° and 30°, preferably less than 20°.

12. Respiratory mask according to anyone of the preceding claims, **characterized in that** it is a facial mask.

13. Assembly for delivering a gas to a patient comprising a gas delivery device, such as a medical ventilator, and a respiratory mask according to anyone of the preceding claims.

## Patentansprüche

1. Atemmaske (1), umfassend:
- einen Maskenkörper (2), der einen Gaseinlass (3) in Fluidverbindung mit einer inneren Kammer (4) umfasst, wobei der Gaseinlass (3) einen inneren Rand (25) umfasst,
- eine vordere Abdeckung (5), die eine mittlere Öffnung (9) und einen nach oben ragenden oberen Arm (6) umfasst, der Kopfgeschirrverbindungsstrukturen (15) umfasst, und
- einen rohrförmigen Winkelverbinder (10), der ein Maskenverbindungsende (23) umfasst, das in den Gaseinlass (3) des Maskenkörpers (2) eingesteckt ist, wobei der Winkelverbinder (10) einen inneren Durchgang (22) in Fluidverbindung mit der inneren Kammer (4) des Maskenkörpers (2) umfasst,
wobei:
- das Maskenverbindungsende (23) des Winkelverbinders (10) und der innere Rand (25) des Gaseinlasses (3) des Maskenkörpers (2) so ausgestaltet sind, dass das Maskenverbindungsende (23) des Winkelverbinders (10) in den Gaseinlass (3) des Maskenkörpers (2) schwenkt, und
- die vordere Abdeckung (5) an dem Winkelverbinder (10) befestigt ist, so dass die mittlere Öffnung (9) der vorderen Abdeckung (5) koaxial zu dem Gaseinlass (3) des Maskenkörpers (2) und zu dem Maskenverbindungsende (23) des Winkelverbinders (10) angeordnet ist,
**dadurch gekennzeichnet, dass**:
- der Winkelverbinder (10) eine ringförmige Nut (28) umfasst, und die vordere Abdeckung (5) einen ringförmigen Rand (27) umfasst, wobei der ringförmige Rand (27) der vorderen Abdeckung (5) in die ringförmige Nut (28) des Winkelverbinders (10) eingeführt wird, wenn die vordere Abdeckung (5) an dem Winkelverbinder (10) befestigt wird,
- der Winkelverbinder (10) ferner einen ersten Anschlag (29) umfasst und die vordere Abdeckung (5) ferner eine zweiten Anschlag (30) umfasst, wobei der zweite Anschlag (30) an den ersten Anschlag (29) des Winkelverbinders (10) anschlägt, wenn die vordere Abdeckung (5) an dem Winkelverbinder (10) befestigt wird und der ringförmige Rand (27) der vorderen Abdeckung (5) in die ringförmige Nut (28) des Winkelverbinders (10) eingeführt wird, und
- ein federndes Element (7) zwischen dem Maskenkörper (2) und einem oberen Arm (6) der vorderen Abdeckung (5) angeordnet ist, so dass das federnde Element (7) zwischen dem Maskenkörper (2) und dem oberen Arm (6) zusammengedrückt wird, wenn der obere Arm (6) zu dem Maskenkörper (2) hin schwenkt.

2. Atemmaske nach dem vorhergehenden Anspruch, **dadurch gekennzeichnet, dass** die äußere Umfangsfläche (26) des Maskenverbindungsendes (23) des Winkelverbinders (10) und der innere Rand (25) des Gaseinlasses (3) zusammen einen Kugelgelenkmechanismus (25, 26) bilden.

3. Atemmaske nach einem der vorhergehenden Ansprüche, **dadurch gekennzeichnet, dass** das federnde Element (7) an der äußeren Umfangswand (8) des Maskenkörpers (2) angeordnet ist.

4. Atemmaske nach einem der vorhergehenden Ansprüche, **dadurch gekennzeichnet, dass** der Gaseinlass (3) des Maskenkörpers (2) ein Bundelement (11) umfasst, das um den Gaseinlass (3) herum angeordnet ist und nach außen vorragt, wobei das Bundelement (11) mindestens einen Teil des inneren Rands (25) umfasst.

5. Atemmaske nach einem der vorhergehenden Ansprüche, **dadurch gekennzeichnet, dass** das Bundelement (11) eine innere bogenförmige Wand (31) umfasst, die mindestens einen Teil des inneren Rands (25) des Gaseinlasses (3) des Maskenkörpers (2) umfasst.

6. Atemmaske nach einem der vorhergehenden Ansprüche, **dadurch gekennzeichnet, dass** die vordere Abdeckung (5) ferner zwei seitliche Arme (12) umfasst, die seitlich vorragen.

7. Atemmaske nach einem der vorhergehenden Ansprüche, **dadurch gekennzeichnet, dass** die beiden seitlichen Arme (12) Kopfgeschirrverbindungsstrukturen (15) umfassen, wobei vorzugsweise ein Kopfgeschirr an den Kopfgeschirrverbindungsstrukturen (15) der vorderen Abdeckung (5) befestigt ist.

8. Atemmaske nach einem der vorhergehenden Ansprüche, **dadurch gekennzeichnet, dass** die vordere Abdeckung (5), die den nach oben vorragenden Arm (6) und/oder die beiden seitlichen Arme umfasst, aus mindestens einem Polymermaterial hergestellt ist.

9. Atemmaske nach einem der Ansprüche 1 bis 7, **dadurch gekennzeichnet, dass** sie ferner ein flexibles Kissen (16) umfasst, das an dem Maskenkörper (2) befestigt ist.

10. Atemmaske nach einem der vorhergehenden Ansprüche, **dadurch gekennzeichnet, dass** die vordere Abdeckung (5) integral an dem hohlen Verbinder (10) gehalten ist.

11. Atemmaske nach einem der vorhergehenden Ansprüche, **dadurch gekennzeichnet, dass** das Maskenverbindungsende (23) des Winkelverbinders (10) und der innere Rand (25) des Gaseinlasses (3) des Maskenkörpers (2) so ausgestaltet sind, dass sie ein Schwenken des Maskenverbindungsendes (23) des Winkelverbinders (10) in den Gaseinlass (3) des Maskenkörpers (2) mit einem Winkel α von zwischen 0° und 30°, vorzugsweise weniger als 20°, gestatten.

12. Atemmaske nach einem der vorhergehenden Ansprüche, **dadurch gekennzeichnet, dass** sie eine Gesichtsmaske ist.

13. Anordnung zur Verabreichung eines Gases an einen Patienten, umfassend eine Gasverabreichungsvorrichtung wie ein medizinisches Beatmungsgerät und eine Atemmaske nach einem der vorhergehenden Ansprüche.

## Revendications

1. Masque respiratoire (1), comprenant :
- un corps de masque (2) comprenant une entrée de gaz (3) en communication fluidique avec une chambre intérieure (4), ladite entrée de gaz (3) comprenant une bordure intérieure (25),
- un flasque frontal (5) comprenant une ouverture centrale (9) et un bras supérieur (6) faisant saillie vers le haut, comprenant des structures (15) de connexion de casque, et
- un connecteur coudé tubulaire (10) comprenant une extrémité de connexion de masque (23) branchée dans l'entrée de gaz (3) du corps de masque (2), ledit connecteur coudé (10) comprenant un passage intérieur (22) en communication fluidique avec la chambre intérieure (4) du corps de masque (2),
dans lequel :
- l'extrémité de connexion de masque (23) du connecteur coudé (10) et la bordure intérieure (25) de l'entrée de gaz (3) du corps de masque (2) sont configurées de telle sorte que l'extrémité de connexion de masque (23) du connecteur coudé (10) pivote dans l'entrée de gaz (3) du corps de masque (2), et
- le flasque frontal (5) est fixé au connecteur coudé (10) de telle sorte que l'ouverture centrale (9) du flasque frontal (5) soit disposée coaxialement avec l'entrée de gaz (3) du corps de masque (2) et avec l'extrémité de connexion de masque (23) du connecteur coudé (10) ,
**caractérisé en ce que** :
- le connecteur coudé (10) comprend une gorge annulaire (28), et
- le flasque frontal (5) comprend un rebord annulaire (27), ledit rebord annulaire (27) du flasque frontal (5) étant inséré dans la gorge annulaire (28) du connecteur coudé (10) lorsque le flasque frontal (5) est fixé au connecteur coudé (10),
- le connecteur coudé (10) comprend en outre une première butée (29) et le flasque frontal (5) comprend en outre une deuxième butée (30), ladite deuxième butée (30) butant contre ladite première butée (29) du connecteur coudé (10) lorsque le flasque frontal (5) est fixé au connecteur coudé (10) et que le rebord annulaire (27) du flasque frontal (5) est inséré dans la gorge annulaire (28) du connecteur coudé (10), et
- un élément élastique (7) est agencé entre le corps de masque (2) et un bras supérieur (6) du flasque frontal (5) de telle sorte que ledit élément élastique (7) soit comprimé entre le corps de masque (2) et le bras supérieur (6) lorsque le bras supérieur (6) pivote vers le corps de masque (2).

2. Masque respiratoire selon la revendication précédente, **caractérisé en ce que** la surface périphérique extérieure (26) de l'extrémité de connexion de masque (23) du connecteur coudé (10) et la bordure intérieure (25) de l'entrée de gaz (3) forment ensemble un mécanisme à rotule (25, 26) .

3. Masque respiratoire selon l'une quelconque des revendications précédentes, **caractérisé en ce que** l'élément élastique (7) est agencé sur la paroi extérieure périphérique (8) du corps de masque (2) .

4. Masque respiratoire selon l'une quelconque des revendications précédentes, **caractérisé en ce que** l'entrée de gaz (3) du corps de masque (2) comprend un élément de collier (11) agencé autour de l'entrée de gaz (3) et faisant saillie vers l'extérieur, ledit élément de collier (11) comprenant au moins une partie de la bordure intérieure (25) .

5. Masque respiratoire selon l'une quelconque des revendications précédentes, **caractérisé en ce que** l'élément de collier (11) comprend une paroi arquée intérieure (31) comprenant au moins une partie de la bordure intérieure (25) de l'entrée de gaz (3) du corps de masque (2).

6. Masque respiratoire selon l'une quelconque des revendications précédentes, **caractérisé en ce que** le flasque frontal (5) comprend en outre deux bras latéraux (12) faisant saillie latéralement.

7. Masque respiratoire selon l'une quelconque des revendications précédentes, **caractérisé en ce que** les deux bras latéraux (12) comprennent des structures de connexion de casque (15), de préférence un casque est fixé aux structures de connexion de casque (15) du flasque frontal (5).

8. Masque respiratoire selon l'une quelconque des revendications précédentes, **caractérisé en ce que** le flasque frontal (5) comprenant le bras supérieur (6) faisant saillie vers le haut et/ou les deux bras latéraux est fabriqué à partir d'au moins un matériau polymère.

9. Masque respiratoire selon l'une quelconque des revendications 1 à 7, **caractérisé en ce qu'**il comprend en outre un coussin flexible (16) fixé au corps de masque (2).

10. Masque respiratoire selon l'une quelconque des revendications précédentes, **caractérisé en ce que** le flasque frontal (5) est maintenu intégralement sur le connecteur creux (10).

11. Masque respiratoire selon l'une quelconque des revendications précédentes, **caractérisé en ce que** l'extrémité de connexion de masque (23) du connecteur coudé (10) et la bordure intérieure (25) de l'entrée de gaz (3) du corps de masque (2) sont configurées pour permettre un pivotement de l'extrémité de connexion de masque (23) du connecteur coudé (10) dans l'entrée de gaz (3) du corps de masque (2) suivant un angle α compris entre 0° et 30°, de préférence inférieur à 20°.

12. Masque respiratoire selon l'une quelconque des revendications précédentes, **caractérisé en ce qu'**il s'agit d'un masque facial.

13. Ensemble d'administration d'un gaz à un patient, comprenant un dispositif d'alimentation en gaz tel qu'un ventilateur médical, et un masque respiratoire selon l'une quelconque des revendications précédentes.
